(19) Europäisches Patentamt
European Patent Office
Office européen des brevets

(11) EP 0 989 838 B1

(12) EUROPEAN PATENT SPECIFICATION

(45) Date of publication and mention
of the grant of the patent:
**19.12.2001 Bulletin 2001/51**

(51) Int Cl.⁷: **A61F 13/15**

(86) International application number:
**PCT/US98/12517**

(21) Application number: **98930280.7**

(22) Date of filing: **15.06.1998**

(87) International publication number:
**WO 98/57609 (23.12.1998 Gazette 1998/51)**

(54) **INTERLABIAL DEVICE ADAPTED FOR MENSES COMPONENT MANAGEMENT**

INTERLABIALE VORRICHTUNG FÜR MENSTRUATIONSBEHANDLUNG

DISPOSITIF INTERLABIAL CONCU POUR ABSORBER LES DIVERS CONSTITUANTS DES MENSTRUES

(84) Designated Contracting States:
**BE DE ES FR GB GR IT NL SE**

(30) Priority: **16.06.1997 US 876206**

(43) Date of publication of application:
**05.04.2000 Bulletin 2000/14**

(73) Proprietor: **THE PROCTER & GAMBLE COMPANY**
**Cincinnati, Ohio 45202 (US)**

(72) Inventors:
• **OSBORN, Thomas, W., III**
  **Cincinnati, OH 45220 (US)**
• **HAMMONS, John, Lee**
  **Hamilton, OH 45011 (US)**
• **HORNEY, James, Cameron**
  **Cincinnati, OH 45211 (US)**

(74) Representative:
**Kremer, Véronique Marie Joséphine et al**
**Procter & Gamble European Service GmbH**
**Sulzbacher Strasse 40**
**65824 Schwalbach am Taunus (DE)**

(56) References cited:
**WO-A-96/07379          US-A- 5 039 431**

**Description**

FIELD OF THE INVENTION

This invention relates to absorbent devices, and more particularly to an interlabial absorbent device, adapted to manage the various components of menses in a manner that makes more effective use of the absorbent capacity of the device. Such devices are typically used by female wearers for catamenial purposes.

BACKGROUND OF THE INVENTION

The development of highly absorbent articles for blood and blood-based fluids such as catamenial products (e.g., sanitary napkins, tampons, or interlabial devices), wound dressings, bandages and surgical drapes can be challenging. Compared to water and urine, blood and blood based fluids such as menses are relatively complex mixtures of dissolved and undissolved components (e.g., erythrocytes or red blood cells). In particular, blood-based fluids such as menses are much more viscous than water and urine due to undissolved components and high viscosity mucosal components. This higher viscosity hampers the ability of conventional absorbent materials to efficiently and rapidly transport these blood-based fluids to regions remote from the point of initial discharge. For example, undissolved components of these blood-based fluids can potentially clog the capillaries of these absorbent materials and high viscosity mucosal components can significantly slow absorption rate. This makes the design of appropriate absorbent systems for blood-based fluids such as menses particularly difficult.

In the case of catamenial products, women have come to expect a high level of performance in terms of comfort and fit, retention of fluid, and minimal staining. Above all, leakage of fluid from the pad onto undergarments is regarded as totally unacceptable. Improving the performance of such catamenial products continues to be a formidable undertaking, although a number of improvements have been made in both catamenial structures, and materials used in such structures. However, eliminating leakage, particularly along the inside of the thighs, without compromising fit and comfort, has not always met the desired needs of the consumer.

Sanitary napkins of the current art have attempted to address absorption of high viscosity bodily fluids, such as menses in various ways. For example, U.S. Patent 4,973,325, issued to Sherrod, et al. on November 27, 1990, provides a transfer member said to facilitate movement of body fluids downward and outward to distant areas of the absorbent. The transfer member, which is positioned above, between and beneath the absorbents, comprises a material intended to readily transfer fluids as well as give up fluid to a cellulosic absorbent. While such a structure may represent an improvement in fluid distribution, the ability to facilitate movement of fluids to distant areas of an absorbent may require a porous network with a pore size distribution sufficiently small (for capillary wicking)l that it can become clogged by the solid components of menses. In another example, U.S. Patent application Serial No. 08/382,921, filed in the name of Horney, et al. on February 3, 1995 and published as PCT Application WO 96/23474 on August 8, 1996, describes an absorbent article having an acquisition component having a pore size in the range of red blood cells, suspended solids, and the like. The acquisition component traps and stores (i.e. filters) such components from the bodily exudate allowing lower viscosity components to pass through for storage in a small pore storage component. Filtering bodily fluids, such as menses, has clear advantages in making use of absorbent capacity. However, the act of filtering a component means that the pores that filtered the component are no longer available for rapid absorption or transport of fluids (i.e. that pore is clogged). However, clogging is of less importance for an externally worn device, such as that described in the Horney, et al. application, than it would be for a device fitting close to a source of bodily fluids, such as an interlabial product, because externally worn devices are typically larger than internally worn devices and bodily fluids can flow to an unclogged portion for absorption.

Interlabial pads have the potential to provide a significantly reduced risk of leakage because they are positioned in close proximity to the vaginal introitus enabling them to intercept bodily fluids such as menses almost immediately after discharge. Numerous attempts have been made in the past to produce an interlabial pad which would combine the best features of tampons and sanitary napkins while avoiding at least some of the disadvantages associated with each of these types of devices. Examples of such devices are described in U.S. Patent 2,917,049 issued to Delaney on December 15, 1959, U.S. Patent 3,420,235 issued to Harmon on January 7, 1969, U.S. Patent 4,595,392 issued to Johnson, et al. on June 17, 1986, and U.S. Patents 5,074,855 and 5,336,208 issued to Rosenbluth, et al. on December 24, 1991 and August 9, 1994 respectively, and U.S. Patent 5,484,429 issued to Vukos, et al. on January 16, 1996. A commercially available interlabial device is FRESH 'N FIT® Padette which is marketed by Athena Medical Corp. of Portland, OR and described in U.S. Patents 3,983,873 and 4,175,561 issued to Hirschman on October 5, 1976 and November 27, 1979, respectively.

However, many of these devices have not met with great commercial success. There are drawbacks associated with all of the above products. For example, the device described in the Delaney patent does not appear to be capable of an easy and comfortable insertion, due to the possibility of the layers of absorbent material opening up during insertion. The commercially available Padette product suffers from the disadvantage that they may not provide protection when a wearer squats.

Further, capillaries on or near the surface of the of such products that is presented to the body may not be able to rapidly absorb the viscous components of bodily exudates, such as menses, or the surface capillaries may become clogged with particulate material, such as cellular debris. Such clogging can reduce absorption efficiency and result in increased risk of leakage.

Thus, a need exists for an interlabial device that is small in size and that can be easily inserted and that provides protection against incontinence, menstrual discharges, and discharges of bodily exudates throughout a wide range of wearer motions.

A need further exists for an interlabial device that resists clogging by components of bodily exudates, such as viscous fluids and cellular debris. Clogging of surface capillaries is not a significant issue for catamenial devices of the current art that are not worn interlabially, such as sanitary napkins, because fluid can spread over their relatively large surface area bypassing any clogged portions. That is, if a small portion of the surface of a sanitary napkin becomes clogged by cellular debris or the like, bodily fluid can readily move to an unclogged area for absorption. Also, catamenial devices of the current art frequently do not have sustained bodily fit so the device can move relative to the body. As a result, bodily exudates may be deposited on one portion of the device at one time and on another portion of the device at another time, reducing the risk of clogging a given portion. On the other hand, an interlabial device is held in relatively the same position with respect to a wearer's vaginal introitus and urethral opening throughout a full range of wearer motions. As a result, bodily exudates are continually deposited on the same portion of the interlabial device. This means the risk of clogging may be higher for an interlabial device when compared to other catamenial devices of the current art.

Therefore, it is an object of the present invention to provide an interlabial absorbent device that is small in size and is easy to insert.

It is another object of the present invention to provide an interlabial absorbent device that consistently blocks both the vaginal introitus and the urethral opening so that it provides protection against menstrual discharges, incontinence, and discharges of bodily exudates throughout a wide range of wearer motions.

It is still another object of the present invention to provide an interlabial absorbent device that "manages" the absorption of high viscosity or insoluble components of bodily fluids such as menses by resisting capillary clogging by such components.

These and other objects of the present invention will become more readily apparent when considered in reference to the following description and when taken in conjunction with the accompanying drawings.

SUMMARY OF THE INVENTION

This invention relates to absorbent devices, and, more particularly, to an absorbent device that is insertable into the interlabial space of a female wearer for catamenial purposes, incontinence protection, or both.

The interlabial absorbent device of the present invention comprises a main absorbent portion and a pair of flexible extensions joined to the main absorbent portion. The main absorbent portion comprises an acquisition member that can rapidly absorb bodily fluids, such as menses, without becoming clogged with solid or high viscosity components of such fluids. The main absorbent portion further comprises a storage member that is in effective fluid contact with the acquisition member. The storage member has a pore volume distribution which enables it to draw absorbed bodily fluids from the acquisition member. The interlabial device further comprises a pair of flexible extensions which are joined to the main absorbent portion adjacent a proximal surface thereof. The proximal surface is that surface of the main absorbent portion that is positioned furthest inward into the space between the wearer's labia when the interlabial device is inserted. Upon insertion, the flexible extensions contact and tend to adhere to the walls of a wearer's labia. The flexible extensions are also preferably capable of covering the wearer's fingertips as the absorbent device is inserted into the interlabial space of the wearer.

BRIEF DESCRIPTION OF THE DRAWINGS

While the specification concludes with claims particularly pointing out and distinctly claiming the subject matter which is regarded as forming the present invention, it is believed that the invention will be better understood from the following description taken in conjunction with the accompanying drawings, in which:

Figure 1 is a perspective view of a preferred embodiment of the interlabial absorbent device of the present invention.

Figure 2 is an end view of the absorbent device shown in Figure 1.

Figure 3 is a perspective view of an alternative preferred embodiment of the interlabial absorbent device of the present invention.

Figure 4 is an end view of an alternative preferred embodiment of the present invention having a pleated main absorbent portion.

Figure 5 is an end view of an alternative embodiment of the pleated embodiment shown in Figure 4.

Figure 6 is a preferred embodiment of the present invention showing a main absorbent portion having a multiple layer structure.

Figure 7 is a cross-sectional view of a wearer's body surrounding and including the wearer's labia majora and labia minora showing the flexible extensions of the present invention covering the wearer's fingertips as the absorbent device of the present invention is inserted into the wearer's interlabial space.

Figure 8 is a cross-sectional view of the same region of the wearer's body shown in Figure 7 showing how the interlabial absorbent device of the present invention fits when the wearer is standing.

## DETAILED DESCRIPTION OF THE INVENTION

### General Description of the Interlabial Absorbent Device

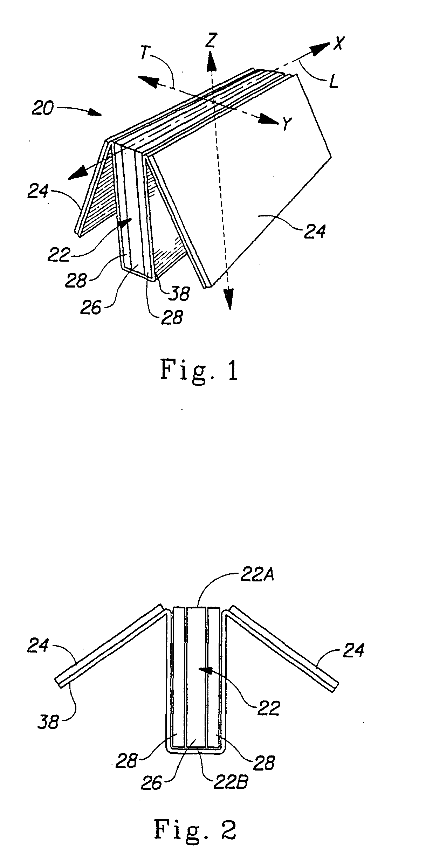

The present invention is directed to an interlabial absorbent device. Figure 1 shows one preferred embodiment of the interlabial absorbent device of the present invention, interlabial device 20. The present invention, however, can be in many other forms, and is not limited to a structure having the particular configuration shown in the drawings.

As used herein the term "interlabial absorbent device" refers to a structure which has at least some absorbent components, and is specifically configured to reside at least partially within the interlabial space of a female wearer during use. Preferably, at least about 30% of the volume of interlabial absorbent device 20 of the present invention resides within such interlabial space when the device is worn, more preferably at least about 50 % of the volume resides within such interlabial space, still more preferably substantially the entire volume of interlabial absorbent device 20 resides within a wearer's interlabial space, and most preferably the entire interlabial absorbent device 20 resides within the interlabial space of a female wearer during use.

As used herein, the term "interlabial space" refers to that space in the pudendal region of the female anatomy which is located between the inside surfaces of the labia majora extending into the vestibule. Located within this interlabial space are the labia minor, the vestibule and the principal urogenital members including the clitoris, the orifice of the urethra, and the orifice of the vagina. Standard medical authorities teach that the vestibule refers to the space bounded laterally by the inside surfaces of the labia minora and extending interiorly to the floor between the clitoris and the orifice of the vagina. Therefore, it will be recognized that the interlabial space as defined above may refer to the space between the inside surfaces of the labia majora, including the space between the inside surfaces of the labia minora also known as the vestibule. The interlabial space for purposes of the present description does not extend substantially beyond the orifice of the vagina into the vaginal interior.

The term "labia" as used herein refers generally to both the labia majora and labia minora. The labia terminate anteriorly and posteriorly at the anterior commissure and the posterior commissure, respectively. It will be recognized by those skilled in the art that there is a wide range of variation among women with respect to the relative size and shape of labia majora and labial minora. For purposes of the present description, however, such differences need not be specifically addressed. It will be recognized that the disposition of the interlabial absorbent device into the interlabial space of a wearer as defined above will require placement between the inside surfaces of the labia majora without regard to the precise location of the boundary between the labia majora and the labia minora for a particular wearer. For a more detailed description of this portion of the female anatomy, attention is directed to *Gray's Anatomy*, Running Press 1901 Ed. (1974), at 1025-1027.

As used herein the term "solid component of bodily fluids" refers to particles having an effective radius of greater than about 5 microns.

The interlabial absorbent device 20 shown in Figure 1 has a longitudinal centerline L which runs along the "x" axis shown in Figure 1. The term "longitudinal", as used herein, refers to a line, axis or direction in the plane of the interlabial device 20 that is generally aligned with (e.g., approximately parallel to) a vertical plane which bisects a standing wearer into left and right body halves when the interlabial device 20 is worn. The terms "transverse," "lateral," " or "y direction" as used herein, are interchangeable, and refer to a line axis or direction that is generally perpendicular to the longitudinal direction. The lateral direction is shown in Figure 1 as the "y" direction and is defined by the lateral centerline "T". The "z" direction, shown in Figure 1, is a direction perpendicular to the plane formed by the longitudinal centerline L and the lateral centerline T. The term "upper" refers to an orientation in the z-direction toward the wearer's head. "Lower" or downwardly is toward the wearer's feet.

As shown in Figure 1, the interlabial device 20 comprises a main absorbent portion (or "central absorbent") 22, and a pair of flexible extensions 24 joined to the main absorbent portion 22. The main absorbent portion 22 should be at least partially absorbent. The main absorbent portion 22 comprises an acquisition member 26 disposed along the longitudinal centerline of the interlabial device 20 and a storage member 28 disposed laterally outboard of at least one of the sides of the acquisition member 26. Preferably, the storage member 28 comprises two portions with one portion being disposed laterally outboard of each of the sides (L, z plane) of the acquisition member 26. The flexible extensions 24 are joined to the main absorbent portion 22 adjacent the proximal end 22A thereof. In use, the proximal end 22A is positioned furthest inward into the wearer's interlabial space. In the preferred embodiment shown in Figure 1, the interlabial device 20 also comprises a liquid impervious backsheet 38 to prevent absorbed exudates from leaking out of the main absorbent portion 22.

The interlabial device 20 should be of a suitable size and shape that allows at least a portion thereof to fit comfortably within the wearer's interlabial space covering a wearer's vaginal orifice, and preferably also the wearer's urethral opening. The interlabial device 20 at least partially blocks, and more preferably completely blocks and intercepts the flow of menses, urine, and other bodily exudates from the wearer's vaginal orifice and urethral opening.

The size of the interlabial device 20 is also important to the comfort associated with wearing the device. In the preferred embodiment shown in Figure 1, the main absorbent portion 22 of the interlabial device 20 has a length as measured along the longitudinal centerline, L, of between about 35 mm and about 120 mm. Preferably, the length of the interlabial device 20 is between about 40 mm and about 100 mm, and more preferably, is between about 45 mm and about 90 mm. The dry caliper (or width) of the main absorbent portion 22 of the interlabial device, as measured in the lateral direction (or "y"-direction) under a confining pressure of 0.25 pounds per square inch (1.7 kPa), is preferably less than or equal to about 15 mm, more preferably the dry caliper is less than about 10 mm. Still more preferably, the dry caliper is less than about 8 mm. A method for dry caliper measurement is provided in the TEST METHODS section below. The depth (or "z"-direction dimension) of the main absorbent portion 22 is preferably between about 8 mm and about 35 mm, and more preferably about 20 mm.

The interlabial device 20 is preferably provided with sufficient absorbency to absorb and retain the exudates discharged from the wearer's body. The capacity of the product, however, is dependent at least partially upon the physical volume of the interlabial absorbent device 20, particularly the main absorbent portion 22 thereof. The main absorbent portion 22 preferably has a capacity of at least about 1 gram of sheep's blood as measured according to the Absorbent Capacity test described in the TEST METHODS section below, and may have a capacity of up to about 30 g by using absorbent gels or foams that expand when wet. Capacities may typically range from about 3 to about 12 grams. Those skilled in the art will recognize that, although they are related, the capacity for absorption of bodily exudates such as menses will typically differ somewhat from the absorbent capacity for sheep's blood. Since the interlabial space can expand, larger volumes can be stored in the interlabial space, if the fluid is stored as a gel, which adjusts to the body pressures. Additionally, if the interlabial absorbent device 20 does not reside completely within the wearer's interlabial space, some of the absorbed exudates may be stored externally to the wearer's interlabial space.

The Main Absorbent Portion

In the preferred embodiment of the interlabial absorbent device 20 shown in Figures 1 and 2, the main absorbent portion 22 comprises an acquisition member 26 and a storage member 28. The acquisition member 26 rapidly acquires bodily exudates, such as menses or urine, before passing them on to the storage member 28 which absorbs and retains the bodily exudates. Preferably, the acquisition member 26 has a structure tailored to rapid acquisition of such bodily fluids and the storage member 28 has a structure tailored to "drawing" at least a portion of the acquired fluids from the acquisition member 26 for storage therein. While such structures will be discussed in detail below, the acquisition member 26 for one preferred embodiment of the present invention comprises a fibrous assembly having a first density and the storage member 28 comprises a fibrous assembly having a second, higher density. The relative densities or the acquisition member 26 and the storage member 28 are such that the acquisition member 26 can rapidly acquire bodily fluids that comprise undissolved components (e. g. cellular debris) and high viscosity components (e. g. mucosal components), as well as more fluid components, without substantial clogging while being able to "give up" at least a portion of such bodily fluids to the storage member 28. In other words, the acquisition member 26 will preferably have a relatively low density (and a relatively large pore volume distribution) and the storage member will have a higher density (and smaller pores).

As is clearly shown in Figures 1 and 2, for the preferred embodiment of the present invention, the acquisition member 26 is disposed along the longitudinal centerline L where it is in a position to acquire such bodily exudates as they are discharged from the vaginal introitus or the urethra. As is also shown in Figures 1 and 2, the storage member 28 of this preferred embodiment comprises two portions, one portion being disposed laterally outboard of each side of the acquisition member 26 (One of skill in the art will recognize that a single portion lying laterally outboard of one side of the acquisition member 26 is also suitable). Each of the portions of the storage member 28 is in effective fluid connection with, and preferably joined to, the acquisition member 26. As used herein, the term "fluid communication" is intended to mean that bodily fluids can be transferred between two elements of the interlabial absorbent device 20 by capillary or other means.

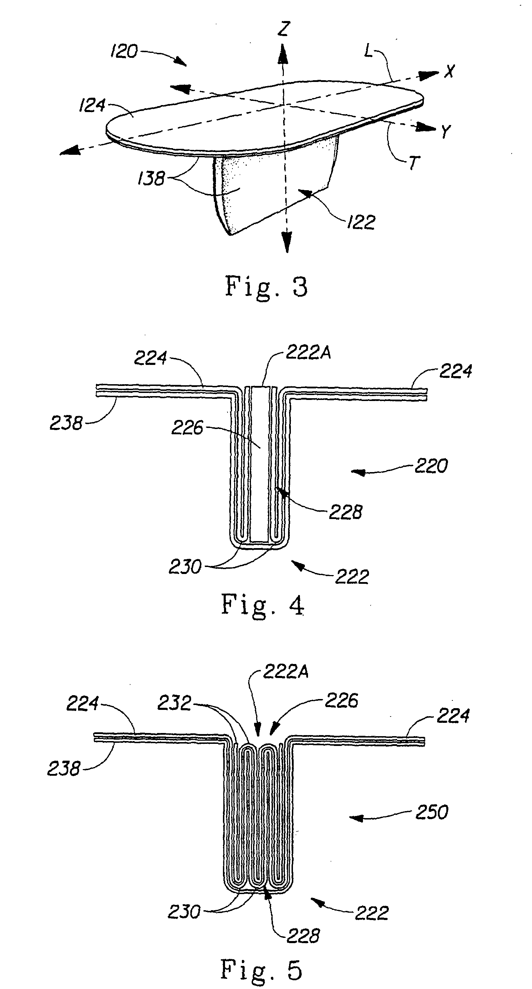

The main absorbent portion 22 may be manufactured in a wide variety of shapes. Non limiting examples include ovoid, trapezoidal, rectangular, rounded rectangular, triangular, cylindrical, hemispherical or any combination of the above. A rounded rectangular shape is particularly preferred because such a- shape eliminates sharp corners that may be uncomfortable. Preferably, as is shown in Figure 1, the main absorbent portion 22 extends the entire longitudinal length of the interlabial absorbent device 20. In an alternative preferred embodiment of the present invention, the main absorbent portion 22 is centered about the lateral centerline T and extends only a portion of the longitudinal length of the flexible extensions 24. Such an embodiment is shown as 120 in Figure 3 and discussed in greater detail in the Alternative Embodiments section below.

Acquisition Member

The acquisition member 26 receives bodily fluids (e. g. menses) as such fluids are released and rapidly acquires them. The acquisition member 26 of the present invention is particularly able to acquire such bodily fluids over substantially the entire wear cycle of the interlabial absorbent device 20 without surface clogging caused by solid or high viscosity components of such fluids that would interfere with such rapid acquisition.

In order to enable such rapid acquisition without clogging, the acquisition member 26 has larger pores relative to the pores of the storage member 28 such that the acquisition member 26 can acquire bodily exudates without being clogged by the suspended solid or high viscosity components of the exudates while "giving up" (partitioning) the remaining fluid portion of bodily exudates to the storage member 28. For the acquisition member 26 and the storage member 28 to function in this manner, the acquisition member 26 should have a high percentage of pores that are larger than the range of pores the storage member 28. Stated another way, the mean capillary radius of the pore volume distribution of the acquisition member 26 should be larger than the mean capillary radius of the pore volume distribution of the storage member 28. As used herein, the term "capillary radius" is intended to mean the equivalent radius as measured during the desorption step (increasing hydrostatic pressure) according to the Pore Volume Distribution test described in the TEST METHODS section below. To be suitable as an acquisition member 26, a material should have a pore volume distribution such that at least 75 percent of the pores therein have a capillary radius greater than about 20 microns. Preferably, at least 75% of the pores have a capillary radius greater than about 30 microns. Still more preferably at least about 75% of the pores have a capillary radius greater than about 50 microns. Particularly preferred materials have a pore volume distribution such that at least 75% of the pores have a capillary radius greater than about 75 microns. The Applicants have found that, as long as the acquisition member 26 has-such a capillary radius or larger, the solid and viscous components of menses do not substantially interfere with the acquisition of such bodily fluids over most of the wear cycle of the interlabial absorbent device 20. As will be discussed in detail below, the smaller pores of the storage member 28 can then acquire the lower viscosity components from the acquisition member 26 because of the higher capillary suction provided by the smaller pores.

The acquisition member 26 is also preferably possesses wet resiliency in order to maintain its ability to acquire solid and viscous components of bodily exudates after the acquisition member has become wet with bodily fluids. As used herein, a material having "wet resiliency" maintains at least about 60 % of its initial wet caliper after being compressed to 50% of its initial wet caliper as described in the TEST METHODS section below. Preferably, the acquisition member 26 comprises a material that has a wet resiliency of at least about 70 %.

Materials that will acquire solid and viscous components of the bodily exudates without substantial clogging include both woven materials, nonwoven materials, absorbent foams; absorbent sponge. Such materials should be either intrinsically hydrophilic or treated to become hydrophilic so water-based bodily fluids can spread rapidly thereon. Suitable woven and nonwoven materials can be comprised of natural fibers (e.g., cellulosic fibers), hydrophilic synthetic fibers (e.g., polymeric fibers, such as polyester, rayon, polypropylene, or polyethylene fibers, that have been treated to be hydrophilic) or from a combination of natural and synthetic fibers. Fibers useful in manufacturing materials suitable for use as an acquisition member 26 preferably have a relatively smooth surface so as to not provide "snags" that can interfere with the rapid absorption of the solid and viscous components of bodily exudates such as menses. When the acquisition member 26 comprises a foam material, the foam can either be a blown foam (e. g., a blown polyurethane foam), a high internal phase emulsion (HIPE) foam, or other foam as may be known to the art.

Suitable cellulosic fibers are the chemically cross-linked and stiffened, twisted (curled) fibers which are discussed more fully in U.S. Patent 4,898,642 to Moore, et al., the disclosure of which is hereby incorporated herein by reference. Such fibers may be formed into a web suitable for use as an acquisition member 26 using air laying or wet laying techniques as are familiar to the art. Preferably, the fibers are air laid as described in commonly assigned U.S. Patent 5,607,414, issued to Richards, et al. on March 4, 1997, the disclosure of which is hereby incorporated herein by reference. Such fibers are preferably thermally bonded by a thermoplastic material as is also described in the aforementioned U.S. Patent 5,607,414.

A suitable foam material for the acquisition member 26 is the HIPE foam described in U.S. Patent 5,563,179, issued to Stone, et al., on October 8, 1996. Also suitable is the polyurethane foam marketed by Foamex Corp. of Eddystone, PA as SIF 100. Such polyurethane foams would require treatment with a suitable surfactant so it would be sufficiently hydrophilic for use as an acquisition member 26.

A preferred material for the acquisition member 26 comprises a needle punched, carded rayon nonwoven material having a basis weight between about 65 grams per square meter and about 75 grams per square meter and a density of between about 0.01 grams per cubic centimeter and about 0.05 grams per cubic centimeter, preferably between about 0.02 grams per cubic centimeter and about 0.04 grams per cubic centimeter. Such a material, with a basis weight of about 69 grams per square meter and a density of about 0.03 grams per cubic centimeter, is available from Sterns Technical Textiles, Inc. of Lockland, OH as material 9290B.

A particularly preferred nonwoven material for the acquisition member 26 comprises a thermally bonded air laid nonwoven material. A suitable thermoplastic staple fiber for such a nonwoven comprises a polyethylene/polypropylene bicomponent material (18 d-tex x 5 millimeter) with a hydrophilic finish and is available from Chisso Corporation of Osaka, Japan. The preferred nonwoven material comprises 100 percent of such thermoplastic staple fibers. The air laying and thermal bonding methods described in the aforementioned U.S. Patent 5,607,414, can be used to form a nonwoven web having a basis weight of about 60 grams per square meter and a dry caliper of about 2.3 millimeters from such fibers. This nonwoven material has been found to have a pore volume distribution such that at least 75% of the pores have a capillary radius greater than about 200 microns.

As can be seen in Figures 1 and 2, the acquisition member 26 is preferably positioned along the longitudinal centerline L of the interlabial absorbent device 20. As can also be seen the acquisition member 26 extends for substantially the entire longitudinal length of the main absorbent portion 22. The depth of the acquisition member 26 is also defined by the depth of the main absorbent portion 22. As is also shown for the preferred embodiment shown in Figures 1 and 2, the acquisition member 26 is disposed between the two portions of the storage member 28. Preferably, the acquisition member has a lateral width (dry caliper) of between about 1.0 millimeter and about 4 millimeters. More preferably, the lateral width is between about 1.5 millimeters and about 2.5 millimeters.

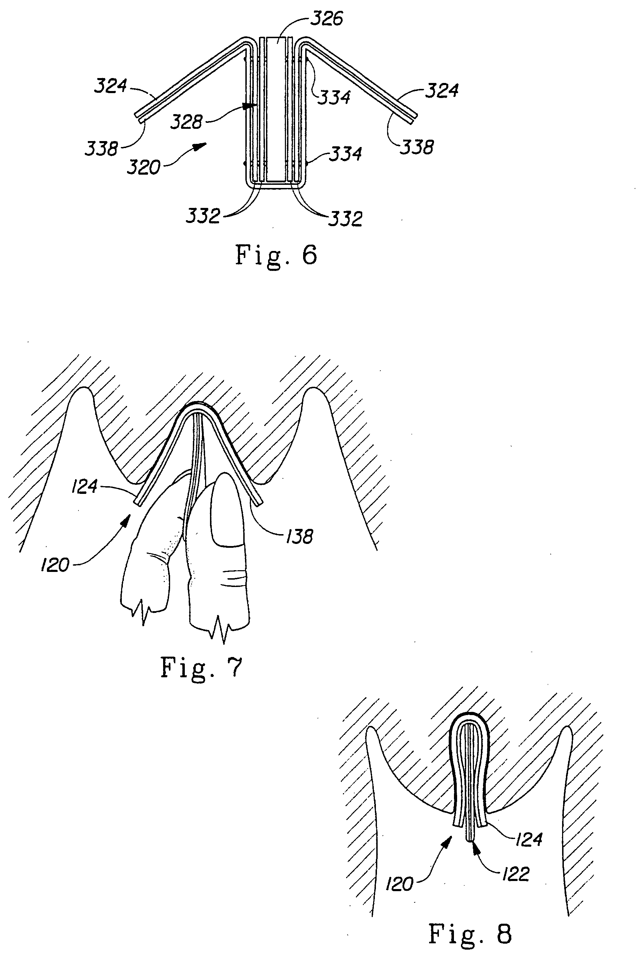

The structure shown in Figures 1 and 2 is particularly advantageous for acquisition and storage of bodily fluids such as menses. As can be seen in Figure 7, when the interlabial absorbent device 20 of the present invention is worm, the main absorbent portion 22 (and, necessarily, the acquisition member 26) has a substantially vertical orientation. This means that gravity and capillary suction cooperate when the acquisition member 26 acquires bodily fluids as they are released. Such cooperation can cause such bodily fluids, particularly the solid and viscous components thereof to be drawn down into the acquisition member 26 and away from the proximal surface 22A of the main absorbent portion. Because such fluids are drawn away from the proximal surface 22A, the risk of clogging any pores of the acquisition member 26 that may lie near that surface is reduced.

The Storage Member

As noted above, the storage member 28 absorbs and retains bodily exudates such as menses or urine, and need not have an absorbent capacity much greater than the total amount of exudate to be absorbed. In particular, the storage member 28 draws fluids from the acquisition member 26 because of the higher capillary suction of the materials comprising the storage member 28. The higher capillary suction of the storage member 28 results from the smaller pores therein. The storage member 28 should have a pore volume distribution wherein the mean capillary radius ranges between about 5 microns and about 50 microns, preferably between about 10 microns and about 40 microns, to provide sufficient capillary suction to draw fluid components of the bodily exudates from acquisition member 26 into the storage member 28.

It is important to insure that the majority of the pores of a storage material are small enough to provide sufficient capillary suction. Such materials preferably have pore volume distributions such that at least about 75% of the pores of the material have a capillary radius of less than about 80 microns. More preferably, such materials have a pore volume distribution such that at least about 75% of the pores of the material have a capillary radius less than about 60 microns. Particularly preferred materials have a pore volume distribution such that at least about 75% of the pores of the material have a capillary radius of less than about 45 microns.

It is also important that the pore volume distribution of a material suitable for use as a storage member 28 be sufficiently large that the pores thereof are not readily clogged by undissolved components of bodily fluids (e. g. cellular debris). Such clogging can block flow through the storage member 28 so that a portion of the storage member 28 may not be available for storage of bodily fluids. Preferably materials used as a storage member 28 have a pore volume distribution such that less than about 10 % of the pores have a capillary radius of less than 5 microns. More preferably, the pore volume distribution is such that less than about 15% of the pores have a capillary radius of less than about 5 microns.

The storage member 28 of the preferred embodiment shown in Figures 1 and 2 may comprise any suitable type of absorbent structure that is capable of absorbing and/or retaining bodily exudates (e.g. menses and/or urine) and of drawing such exudates from the acquisition member. The storage member 28 may be manufactured and from a wide variety of liquid-absorbent materials commonly used in absorbent articles such as comminuted wood pulp which is generally referred to as airfelt. Examples of other suitable absorbent materials include creped cellulose wadding;

meltblown polymers including coform; chemically stiffened, modified or cross-linked cellulosic fibers; synthetic fibers such as crimped polyester fibers; peat moss; tissue including tissue wraps and tissue laminates; absorbent foams; absorbent sponges; superabsorbent materials, such as superabsorbent polymers and absorbent gelling materials; or any equivalent material or combinations of materials, or mixtures of these. The storage member 28 may comprise a single material or a combination of materials, such as a wrapping layer surrounding a central wadding comprised of a different absorbent material.

A suitable combination of materials for the storage member 28 comprises a laminate of a fibrous absorbent gelling material disposed between two plies of air laid tissue. In this embodiment, the absorbent gelling material is provided at a level of between about 0.005 gram per square centimeter and about 0.15 grams per square centimeter, preferably between about 0.007 grams per square centimeter and 0.07 grams per square centimeter. A suitable absorbent gelling material is available from Courtaulds Fibers/Allied Colloids, a joint venture company, West Midlands, England as OASIS and a suitable tissue is an airlaid tissue available from Fort Howard Tissue Company of Green Bay, Wisconsin, and having a basis weight of 35 lbs. per 3000 sq. ft (57 grams per square meter).

Alternatively, an air laid combination of cellulosic fibers as are commonly known to the art and an absorbent gelling material is also suitable for use as a storage member 28. For example, the Applicants have found that such an air laid combination having a basis weight of 0.025 grams per square centimeter and comprising about 17% Fiberdri 1160 available from Camelot Superabsorbents, Ltd. of Calgary, Alberta, Canada is particularly suitable.

For designs where use of absorbent gelling materials is undesirable, cotton wadding having a basis weight between about 75 grams per square meter and about 250 grams per square meter and a dry caliper of between about 0.5 millimeters and about 3.0 millimeters is preferred. A particularly preferred cotton wadding, having a basis weight of about 190 grams per square meter and a dry caliper of about 1.3 millimeters, is available from REVCO DS, Inc. of Twinsburg, OH as Cotton Cosmetic Squares (Item 2736).

As is shown in Figures 1 and 2, the storage member 28 for this preferred embodiment of the present invention comprises two portions with one portion lying on either side of the acquisition member 26 and laterally outboard thereof. As can also be seen each portion extends substantially the entire longitudinal length of the main absorbent portion 22 and the full depth thereof. The thickness (dry caliper) of each portion of the storage member 28 is preferably between about 4 percent and about 40 percent of the dry caliper of the main absorbent portion 22. More preferably, the thickness is between about 10 percent and about 30 percent of the dry caliper of the main absorbent portion 22.

Such a structure maximizes the contact area between the acquisition member 26 and the storage member 28. As a result, the interlabial absorbent device 20 of the present invention makes full use of available storage capacity. As was discussed with respect to the acquisition member 26, gravity and capillary suction can cooperate to draw bodily fluids down into the acquisition member 26. Since the preferred structure shown in Figures 1 and 2 provides for vertically oriented contact between the acquisition member 26 and the storage member 28 throughout one major face of the acquisition member 26, fluids that have been drawn deep into the acquisition member 26 can be drawn into the storage member 28 at the same depth. This means that full utilization of the available storage capacity of the storage member 28 does not only depend on lateral flow through the storage member 28 as would be necessary if there were no acquisition member 26. As a result, localized portions of the storage member 28 are less likely to become saturated before the full absorbent capacity of the storage member 28 is utilized.

Flexible Extensions

As shown in Figures 1 and 2, the interlabial absorbent device 20 also comprises a pair of flexible extensions 24 which are joined to main absorbent portion 22 adjacent the proximal surface 22A thereof. In the preferred embodiment shown in Figures 1 and 2, the flexible extensions 24 are generally rectangular in shape. Other shapes are also possible for the flexible extensions 24 such as semicircular, trapezoidal, or triangular. The flexible extensions 24 preferably are from about 40 mm to about 160 mm in length, more preferably from about 45 mm to about 130 mm in length, and most preferably from about 50 mm to about 115 mm in length. While the flexible extensions 24 can have a longitudinal length which is shorter than the main absorbent portion 22, preferably they have a length which is the same as or longer than the main absorbent portion 22. The width of a flexible extension refers to the distance from the attachment of flexible extension 24 to the main absorbent portion 22 (or the proximal end 24A of the flexible extension 24) to the distal end (or free end) 24B of the flexible extension 24. The width of a flexible extension 24 is preferably about equal to or greater than the depth of the main absorbent portion 22 as described above. The dry caliper of the flexible extensions is preferably less than or equal to about 3 mm, more preferably less than or equal to about 2 mm, and most preferably less than or equal to about I mm. Ideally the dry caliper of the flexible extensions 24 and the main absorbent portion 22 are selected such that the dry caliper of the overall absorbent interlabial structure 20 is less than or equal to about 8 mm.

The flexible extensions 24 may be constructed from any material having the requisite flexibility and dry caliper. Suitable materials include: woven and nonwoven materials; polymeric materials such as apertured formed thermoplastic films, apertured plastic films, and hydroformed thermoplastic films; porous foams; reticulated foams; reticulated thermoplastic films; and thermoplastic scrims. Suitable woven and nonwoven materials can be comprised of natural fibers (e.g., wood or cotton fibers), synthetic fibers (e.g., polymeric fibers such as polyester, rayon, polypropylene, or polyethylene fibers) or from a combination of natural and synthetic fibers.

A preferred material for constructing the flexible extensions 24 is a tissue layer. A suitable tissue is an airlaid tissue available from Fort Howard Tissue Company of Green Bay, Wisconsin, and having a basis weight of 35 lbs. per 3000 sq. ft (57 grams per square meter). Another suitable airlaid tissue is available from Merfin Hygienic Products, Ltd., of Delta, British Columbia, Canada, having a basis weight of 61 grams per square meter and having the designation grade number 176. Although not necessary for the operation of the present invention, the flexible extensions 24 are preferably backed by the backsheet 38 as is described below.

In the preferred embodiments shown in Figures 1 and 2 the pair of flexible extensions 24 may comprise separate sheets of material independently joined to the main absorbent portion 22 adjacent the proximal surface 22A thereof. Preferably, the flexible extensions 24 are arranged symmetrically about the longitudinal centerline L of the main absorbent portion 22. More preferably, the flexible extensions 24 are joined to the proximal surface 22A of the main absorbent portion 22 within about 3 mm of that surface.

The term "joined", as used herein, encompasses configurations in which an element is directly secured to another element by affixing the element directly to the other element; configurations in which the element is indirectly secured to the other element by affixing the element to intermediate member(s) which in turn are affixed to the other element; and configurations in which one element is integral with another element; i.e., one element is essentially part of the other element.

Backsheet

The preferred embodiment of the interlabial absorbent device 20 shown in Figures 1 and 2 further comprises a backsheet 38 positioned over and joined to all or a portion of its back surface, including the flexible extensions 24.

The backsheet 38 is preferably impervious or semi-pervious to liquids (e.g., menses and/or urine) and flexible. As used herein, the term "flexible" refers to materials which are compliant and will readily conform to the general shape and contours of the human body. The backsheet 38 prevents the exudates absorbed and contained in the main absorbent portion 22 from wetting articles which contact the interlabial absorbent device 20 such as the wearer's undergarments. The backsheet 38 also assists the main absorbent portion 22 in preventing the wearer's body from being soiled by exudates. Additionally, use of a backsheet may provide an improved (e.g. more sanitary) surface for the wearer to grasp between her fingers as the interlabial absorbent device 20 is inserted, or as the soiled device is optionally removed with the fingers.

The backsheet 38 may comprise a woven or nonwoven material, polymeric films such as thermoplastic films of polyethylene or polypropylene, or composite materials such as a film-coated nonwoven material. Preferably, the backsheet is a polyethylene film having a thickness of from about 0.012 mm (0.5 mil) to about 0.051 mm (2.0 mils). An exemplary polyethylene film is manufactured by Clopay Corporation of Cincinnati, Ohio, under the designation P18-0401. The backsheet 38 may permit vapors to escape from the main absorbent portion 22 (i.e., breathable) while still preventing exudates from passing through the backsheet.

Optional Components

The interlabial device 20 in any of the embodiments shown in the drawings may comprise other optional components.

Topsheet

For example, the interlabial device 20 may comprise a topsheet positioned over and joined to all or a portion of the body facing surface of the device 20. Preferably, if a topsheet is used, it is joined to at least a portion of the main absorbent portion 22. Preferably, a topsheet, when used, is disposed on and joined to both the proximal surface 22A of the main absorbent portion 22 and the body facing surface of the flexible extensions 24.

If a topsheet is used, the topsheet should be compliant, soft feeling, and nonirritating to the wearer's skin. Further, the topsheet should be liquid pervious permitting liquids (e.g., menses and/or urine) to readily penetrate through its thickness. A suitable topsheet may be manufactured from a wide range of materials such as woven and nonwoven materials; polymeric materials such as apertured formed thermoplastic films, apertured plastic films, and hydroformed thermoplastic films; porous foams; reticulated foams; reticulated thermoplastic films; and thermoplastic scrims. Suitable woven and nonwoven materials can be comprised of natural fibers (e.g., wood or cotton fibers), synthetic fibers (e.g., polymeric fibers such as polyester, rayon, polypropylene, or polyethylene fibers) or from a combination of natural and synthetic fibers.

In particular, the topsheet may comprise an apertured formed film. Apertured formed films are pervious to bodily exudates and, if properly apertured, have a reduced tendency to allow liquids to pass back through and rewet the wearer's skin. Thus, the surface of the formed film which is in contact with the body remains dry, thereby reducing body soiling and creating a more comfortable feel for the wearer. Suitable formed films are described in U.S. Patent 3,929,135, which issued to Thompson on December 30, 1975; U.S. Patent 4,324,246, which issued to Mullane, et al. on April 13, 1982; U.S. Patent 4,342,314, which issued to Radel, et al. on August 3, 1982; U.S. Patent 4,463,045, which issued to Ahr, et al. on July 31, 1984; and U.S. 5,006,394, which issued to Baird on April 9, 1991. The preferred topsheet for the present invention is the formed film described in one or more of the above patents and marketed on sanitary napkins by The Procter & Gamble Company of Cincinnati, Ohio as the "DRI-WEAVE" topsheet.

The body contacting surface of a preferred formed film topsheet is also hydrophilic so as to help liquid to transfer through the topsheet faster than if the body contacting surface was not hydrophilic so as to diminish the likelihood that menstrual fluid will flow off the topsheet rather than flowing into and being absorbed by the main absorbent portion 22. In a preferred embodiment of such a topsheet, surfactant is incorporated into the polymeric materials of the formed film topsheet. Alternatively, the body contacting surface of the topsheet can be made hydrophilic by treating it with a surfactant such as is described in U.S. Patent 4,950,254 issued to Osborn.

Body Adhesive

Optionally, the flexible extensions 24 may also be provided with a bio-compatible adhesive to assist the adhesion of the flexible extensions 24 to the inside surfaces of the wearer's labia. The strength of such an adhesive should be selected to assist the interlabial absorbent device 20 in staying in place, while still allowing for reliable, and comfortable removal of the device from the wearer's interlabial space. Suitable adhesives are discussed in U.S. Patent 5,336,208, issued to Rosenbluth, et al. on August 9, 1994, the disclosure of which is incorporated herein by reference.

Alternative Embodiments

Several alternative preferred embodiments of the present invention are also contemplated. Examples of such embodiments are discussed below.

Extended Flexible Extensions

In the alternative embodiment, interlabial device 120, which is shown in Figure 3, the longitudinal length of the main absorbent portion 122 is only a fraction of the longitudinal length of the flexible extensions 124. Such a structure has the advantage having a smaller volume and, as a result, taking up a smaller portion of the volume of a wearer's interlabial space than embodiment shown in Figures 1 and 2, interlabial device 20. Conversely, the main absorbent portion 122 of alternative interlabial device 120 will have a lower overall capacity than the main absorbent portion 22 of interlabial device 20.

Overall, each of the components of alternative interlabial device 120 comprise the same elements as interlabial device 20. However, as noted above the size relationships between the components differ. In particular, the main absorbent portion 122 alternative interlabial device 120 has a smaller longitudinal length than the main absorbent portion 22 of interlabial device 20. Depending on the desired relationship between device volume and absorbent capacity, the main absorbent portion 122 may have a longitudinal length of between about 30% and about 90% of the longitudinal length of the flexible extensions 124. Preferably, the longitudinal length of the main absorbent portion 122 is between about 30% and about 60% of the longitudinal length of the flexible extensions 124.

Alternative embodiments (not shown) wherein the longitudinal length of the flexible extensions is less than the longitudinal length of the main absorbent portion are also contemplated with respect to the present invention. Preferably, such flexible extensions are centered about the transverse centerline of such embodiments and extend in the longitudinal direction for at least about one third of the longitudinal length of the main absorbent portion.

In a variation of this alternative embodiment (not shown), the depth of the main absorbent portion 122 can be greater than the lateral width of one of the flexible extensions 124. Such a structure can provide additional absorbent capacity while compensating for some of the volume loss to the main absorbent portion 122 caused by decreasing the longitudinal length thereof.

Pleated Main Absorbent Portion

In an alternative preferred embodiment, interlabial device 220, shown in Figure 4, the storage member 228 comprises a pleated structure. As shown in Figure 4, the storage member 228 comprises a folded tissue web. The folded tissue web preferably has a strength greater than that of standard non-wet strength toilet tissue. Preferably, the storage member 228 comprises a tissue having a temporary wet strength of greater than or equal to about 50 grams as measured according to the wet burst strength method described in the TEST METHODS section below. Preferably the temporary wet strength is greater than or equal to about 100 g. In a preferred design this wet strength will decay to about 50% or less of the original strength over about 30 minutes.

As shown in Figure 4, the tissue web comprising the storage member 228 is folded into a pleated structure comprising a plurality of pleats 230 that are arranged in a laterally side-by-side relationship. The tissue web can be folded so that it has any suitable number of pleats. Preferably, the tissue web is folded so that the overall dry caliper (i.e., the width) of the storage member 228 of this embodiment is between about 2 mm and less than or equal to about 7 mm.

The pleats 230 in the folded tissue web are preferably connected or joined (or retained) in some suitable manner so that the pleated sections maintain their pleated configuration, and are not able to fully open. The pleats can be connected by a variety of means including the use of thread, adhesives, or heat sealing tissues which contain a thermoplastic material, such as, polyethylene. A preferred design uses stitching which joins all of the pleats 230 in the storage member 228 together. Preferably, the main absorbent structure 222 is provided with five stitch locations (four at the corners and one additional location approximately midway between the two lower corners).

The alternative preferred embodiment shown in Figure 4, preferably has a storage member 228 and flexible extension 224 dimensions similar to those described above for the embodiment shown in Figures 1 and 2. The width of the storage member 228 of the interlabial device 220 as measured in the lateral direction (y-direction) is preferably between about 1 mm and about 2 mm. Preferably, in a preferred embodiment, the width of the main absorbent portion of the interlabial device 20 is about 4.5 mm.

The pleated design shown in Figure 4 has the additional benefit of easily providing the flexible extensions 224. The extensions 224 can comprise the same material as the storage member 228, or they can comprise a different material. The extensions 224 are disposed within about 1 millimeter of or, preferably, adjacent to the proximal surface 222A of the main absorbent portion 222 and joined thereto. More preferably, as is shown in the embodiment of Figure 4, the extensions 224 are integral portions of the storage member 228 (that is, the extensions 224 comprise integral extensions of the absorbent tissue material that is folded to form the storage member 228).

The acquisition member 226 and the flexible extensions 224 of the interlabial absorbent device 20 shown in Figure 4 may be constructed from any of the materials discussed with respect to the equivalent members in the embodiment shown in Figures 1 and 2. Similarly, this alternative preferred embodiment also comprises a backsheet 238 disposed on the lower surface thereof.

In a variation of the structure of Figure 4, which is shown in Figure 5 as interlabial device 250, both the acquisition member 226 and the storage member 228 are pleated. Such a structure has the additional benefit of increased surface contact between the acquisition member 226 and the storage member 228 because the storage pleats 203 and the acquisition pleats 232 are inter meshed. One skilled in the art will recognize that the dry caliper of the main absorbent portion 222 of this alternative embodiment is determined by wearer comfort. As a result, the material comprising the acquisition member 226 of this embodiment is thinner (has a lower dry caliper) than the material comprising the embodiment shown in Figure 4. However, because of the folded nature of the acquisition pleats 232, the proximal surface 222A of interlabial device 250 has a body contact area similar to the body contact area of the proximal surface 222A of interlabial device 220 of Figure 4.

In another variation of the pleated structure shown in Figure 4, the storage member 328 may comprise a plurality of individual layers 332 joined in a face-to-face relationship. Such a device is shown in Figure 6 as interlabial device 320. Interlabial device 320 may have all of the same characteristics described above for the pleated structure 220. One benefit of the use of a plurality of individual layers 332 is that the various layers may comprise different materials with different properties or characteristics. Each of the flexible extensions 324 may be integral with one of the individual layers 332 or may be joined separately to the upper portion 326 of the storage member 328. Preferably, the individual layers 332 are arranged in a side-by-side relationship so that the spaces between the layers are oriented in the z-direction (as shown in Figure 6).

As previously discussed, the interlabial absorbent device 20 of the present invention is preferably designed to be placed entirely within the interlabial space of a wearer. To use the interlabial absorbent device 20 of the present invention, the wearer holds the main absorbent portion 22 between her fingers. As shown in Figure 7, the flexible extensions 24 are spread apart so as to cover the tips of the wearer's fingers during insertion. This feature provides for a hygienic insertion of the interlabial absorbent device 20 of the present invention. The proximal surface 22A is inserted first and furthest into the interlabial space. The wearer may assume a squatting position during insertion to assist in spreading the labial surfaces. Once the interlabial absorbent device 20 is inserted, the flexible extensions 24 tend to adhere to the inside surfaces of the labia. When the wearer is standing, the labial walls close more tightly around the interlabial absorbent device 20 as shown in Figure 8.

The interlabial device 20 is preferably at least partially retained in place by exerting a slight laterally outwardly-oriented pressure on the inner surfaces of the wearer's labia minora, labia majora, or both. Additionally, the product is also held by attraction of naturally moist labial surfaces to the tissue comprising the flexible extensions 24.

The interlabial absorbent device 20 can be worn as a "stand alone" product. Alternatively, it can be worn as a back up to a tampon, or in combination with a sanitary napkin, pantiliner, or incontinence pad for menstrual or incontinence use. If the interlabial absorbent device 20 is used with a sanitary napkin, the sanitary napkin can be of any thickness. Use with a sanitary napkin may be preferred at night to reduce rear soiling. The interlabial device 20 can be worn in conventional panties, or it can be used with menstrual shorts.

Numerous alternative embodiments of the interlabial absorbent device of the present invention are possible. For example, these products are designed to be removed by urination, although an alternative extraction string or loop may be used. These products may also be used with medicinal treatments. These products may be constructed of materials which are biodegradable and/or which will fragment in water with agitation (as in a toilet). The interlabial absorbent device 20 may also be constructed with a plurality of slits in the main absorbent portion 22 so as to permit bending of the product in multiple independent directions. Such a structure allows the product to more easily respond to the stresses associated with body movements. In a preferred version of the embodiment shown in Figure 4, the ends of the surface of the central absorbent facing away from the body may be rounded to reduce the force on the product during sitting. The top surface of the structure may have one or more slits or have other regions of preferred bending so that product may easily adjust to the vertical pressure against the pelvic floor, to help accommodate the nonlinear surface of the pelvic floor between the clitoris and the perineum. The flexible extensions 24 of the absorbent devices described above may also act as a spring in both wet and dry conditions such that the sides of the product tend to expand outward pressing against the lateral walls of the labial vestibule, thereby, holding the product in place. In addition, it is preferred that the flexible extensions 24 maintain the ability to act as a "spring" when wet, such as when the product is saturated with liquid. Structures, such as polyurethane foams can provide these properties.

TEST METHODS

Dry Caliper

Principle

Dry caliper of a sample can be determined using a comparator gauge that is weighted to provide a predetermined confining pressure.

Apparatus

A suitable comparator gauge gage is the Ames, Model 130 with dial indicator Model 482, available from the B. C. Ames, Company of Waltham, MA. The comparator gauge should have a circular comparator foot made of aluminum capable of exerting a loading pressure of 0.25 pounds per square inch (1.7 kPa). It will be recognized that the diameter of the comparator foot can be varied to accommodate different sample sizes as long as the loading pressure remains constant.

Operation

1. The comparator gauge is zeroed according to the manufacturer's instructions.

2. The comparator foot is raised and the sample is placed on the base plate. The sample is positioned on the base plate so that when the foot is lowered it is in the center of the sample. The comparator foot should be at least 5 millimeters from all sample edges. Try to smooth out or avoid any wrinkles in the sample.

3. Gently lower the foot onto the sample.

4. Determine caliper by reading the comparator dial 30 seconds after the foot comes in contact with the sample.

5. Repeat steps 2 through 4 for an additional 2 samples.

Calculations

The average of the three readings is the dry caliper of the material.

Absorbent Capacity

Principle

The total absorbent capacity of the interlabial absorbent device is measured as the difference between the dry and loaded weights of the device where the loading fluid is analogous to menses.

Apparatus

Analytical Balance  Accurate to 0.1 gram

Fluid Container  Glass, able to hold 1 liter with sufficient wall depth to insure no spillage when samples are inserted or removed. The container must also have dimensions greater than 150% of the dimensions of the interlabial absorbent device in the x and y directions.

Filter Paper  Available from Filtration Science Corp., Eaton-Dikeman Division of Mount Holly Springs, PA as paper # 631

Test Fluid  Sterile defibrinated sheep blood which is available from Cleveland Scientific, Inc. of Bath, OH. Sheep's blood is a labile material having a limited shelf life even when stored under appropriate conditions. It comprises a heterogeneous mixture having approximately 15% by weight hemoglobin and 75-80% by weight water with low levels of ionic salts, lactic acid, and urea. The blood should be stored in a sealed container at 4°C±1°C prior to use. It should be discarded after two weeks if it is not used. Testing is carried out at a temperature of 23°C±1°C. The sheep's blood should be warmed in a water bath to that temperature and agitated to insure even distribution of components before being transferred into the fluid container.

Procedure

1) Condition the test samples by leaving them in a room at 50% relative humidity and at 73°F (23°C) for a period of two hours prior to the test. The test should be performed under similar conditions.

2) Pour about 1 liter of sheep's blood into the fluid container.

3) Weigh the test sample to the nearest 0.1 gram to determine the dry weight.

4) Submerge the sample in the sheep's blood.

5) Remove the article from the sheep's blood after 15 minutes.

6) Place the article on a horizontal wire mesh screen having square openings 0.25 inches x 0.25 inches (64 millimeters x 64 millimeters) and allow to drain for 5 minutes.

7) Weigh the sample to the nearest 0.1 gram and record the weight

8) Place the sample between two pieces of the filter paper and apply a uniform load of 0.25 pounds per square inch (1.7 kPa) to the sample for 35 seconds.

9) Weigh the sample to the nearest 0.1 gram and record the weight.

10. Increase the loading to 1 pound per square inch (6.8 kPa) and maintain the pressure for 19 seconds.

11. Remove the sample from between the filter paper and reweigh the sample to the nearest 0.1 gram to determine the loaded weight.

Calculation and Reporting

Absorbent capacity is defined as the difference between the loaded and the dry weights.

Pore Volume Distribution

Principle

Pore volume distribution for a sample is measured down to a pore size of about five microns using the Textile Research Institute (Princeton, NJ) Liquid Porosimeter. This instrument (i) applies pre-selected, generally incremental, hydrostatic air pressures to a sample pad that can absorb/desorb fluid through a fluid-saturated membrane and (ii) determines the incremental and cumulative quantity of fluid that is absorbed/desorbed by the pad at each pressure. A weight is positioned on the sample to ensure good contact between sample and membrane and to apply an appropriate mechanical confining pressure. A fluid having a low surface tension ($\gamma$) is typically used to ensure wettability ($\cos(\theta)$ =1) of fiber surfaces.

Each sample evaluation comprises an absorption/desorption cycle. In the absorption sequence cumulative volume absorbed versus incrementally decreasing hydrostatic pressure is measured. This is followed by a single desorption sequence where cumulative volume desorbed versus incrementally increasing hydrostatic pressure is measured. Hydrostatic pressures range from a high pressure corresponding to an equivalent radius:

$$r = 2 \, \gamma \, \cos(\theta)/P$$

of approximately five microns to a zero or near-zero pressure corresponding to an equivalent radius at least about 1000 microns or greater.

Additional detail is provided by the following references:

1. A. Burgeni and C. Kapur, *Capillary Sorption Equilibria in Fibrous Masses,* Textile Research Journal 37, 356 (1967).

2. H. G. Heilweil, ed., *Determining Pore Sire Distributions in Fibrous Materials,* Notes on Research, Textile Research Institute, Number 363 (April 1984).

3. B. Miller and I. Tyomkin, An Extended Range Liquid Extrusion Method for Determining Pore Size Distribution. Textile Res. J. 56, 35 (1986).

Apparatus

Porosimeter (Available from Textile Research Institute of Princeton, NJ as Model LP-5)

Membrane: MILLIPORE 0.22 $\mu$M pore size GS Filter 90 mm (Available from Millipore Corp. of Bedford, MA as Catalog Number GSWP 090-25)

Porous Plate (Available from Fisher Scientific of Pittsburgh, PA)

Petree Dish Lid - 90 cm diameter

Small soft bristles brush (~0.5" wide)

Test Fluid

Surfactant Solution:  0.2% by weight solution of TRITON X-100 (available from Rohm and Haas, Philadelphia, PA) in deionized water

Procedure

Sample Preparation

Shortly before measuring pore volume distribution, the caliper of the sample is measured (as described herein) under a confining pressure of 0.2 psi (1.4 kPa). Sample density is calculated from the weight, caliper, and area of the sample.

Confining Pressure

A confining pressure of 0.2 psi (1.4 kPa) is used for the pore-volume distribution measurement. The same or equivalent weight that is used for determining sample density is also used for applying the confining pressure during the pore volume measurement.

Instrumentation Configuration

Set up the porosimeter according to the instruction manual.

Absorption/Desorption Cycle Characteristics

An absorption/desorption cycle comprises two steps. First, a single absorption sequence where cumulative volume absorbed versus incrementally decreasing hydrostatic pressure is measured. This is followed by a single desorption sequence where cumulative volume desorbed versus incrementally increasing hydrostatic pressure is measured. Hydrostatic pressures range from a high pressure corresponding to an equivalent radius of approximately five microns to a zero or near-zero pressure corresponding to an equivalent radius at least about 1000 microns or greater.

Absorption/Desorption Procedure for an Initially Dry Sample.

1. Input parameters are entered as described in the equipment instructions and the controlling computer program is started.

2. The top of the instrument test cell is secured and the vent valve is shut. The liquid flow valve to the balance is opened. At this step the sample is not yet in the test cell.

3. The controlling computer program is continued. The hydrostatic pressure adjusts to a pre-set value (i.e., STOP RADIUS) that is slightly higher than the first inputted pressure used in the experiment.

4. After equilibration is signaled, the liquid valve to the balance is shut, the test cell is opened, the sample is positioned on the membrane, the confining weight is positioned on the sample, and the top of the instrument test cell is secured.

5. The program is continued and hydrostatic pressure adjusts to first inputted pressure.

6. After equilibration is signaled, the fluid valve to balance is opened.

7. The sample is cycled through a predetermined series of pressures which correspond to specific equivalent pore radii.

Blank Subtraction

A blank run is recorded, as above, but with the test cell empty. This determines the background absorption/ desorption response of the system and membrane. Values for cumulative absorption and desorption volumes versus hydrostatic pressure are obtained. These values are used to correct the corresponding values measured for the sample.

Calculation of Normalized Cumulative Volumes

Based on the incremental volume values, the controlling computer program calculates blank-corrected values for cumulative volume versus equivalent pore radius. Cumulative volumes are divided by the dry weight of the pad and reported in unites of mm$^3$/mg. Cumulative volumes are divided by the volume at saturation (i.e., the cumulative volume measured for the largest equivalent radius at zero or near-zero pressure) to obtain the normalized cumulative volume (expressed as %) versus equivalent pore radius.

Burst Strength

Overview

The test specimen, held between annular clamps, is subjected to increasing force that is applied by a 0.625 inch diameter, polished stainless steel ball. The burst strength is that force that causes the sample to fail. Burst strength may be measured on wet or dry samples.

Apparatus

| | |
|---|---|
| Burst Tester | Intelect-II-STD Tensile Test Instrument, Cat. No. 1451-24PGB or the Thwing-Albert Burst Tester are both suitable. Both instruments are available from Thwing-Albert Instrument Co., Philadelphia, PA. The instruments must be equipped with a 2000 g load cell and, if wet burst measurements are to be made, the instruments must be equipped with a load cell shield and a front panel water shield. |
| Conditioned Room | Temperature and humidity should be controlled to remain within the following limits: |

| | | |
|---|---|---|
| | Temperature: | 73±3°F (23°C±2°C) |
| | Humidity: | 50±2% Relative Humidity |

| | |
|---|---|
| Paper Cutter | Scissors or other equivalent may be used |
| Pan | For soaking wet burst samples, suitable to sample size |
| Solution | Water for soaking wet burst samples should be equilibrated to the temperature of the conditioned room. |
| Timer | Appropriate for measuring soak time |

Sample preparation

1) Cut the sample to a size appropriate for testing (minimum sample size 4.5 in x 4.5 in). Prepare a minimum of five samples for each condition to be tested.

2) If wet burst measurements are to be made, place an appropriate number of cut samples into a pan filled with temperature-equilibrated water.

Equipment Setup

1) Set the burst tester up according to the manufacturer's instructions. If an Intelect-II-STD Tensile Test Instrument is to be used the following are appropriate:

Speed: 12.7 centimeters per minute

Break Sensitivity: 20 grams

Peak Load: 2000 grams

2) Calibrate the load cell according to the expected burst strength.

Measurement and Reporting

1) Operate the burst tester according to the manufacturer's instructions to obtain a burst strength measurement for each sample.

2) Record the burst strength for each sample and calculate an mean and a standard deviation for the burst strength for each condition.

3) Report the mean and standard deviation for each condition to the nearest gram.

Wet Resiliency

Principle

A test sample, saturated to its free absorbent capacity with 0.9% saline solution, is compressed to one half its precompression caliper. The pressure is then released, and the sample is allowed to recover thickness in the presence of the expelled fluid. The percent recovery is based on the original wet caliper of the uncompressed sample.

Test Fluid

An aqueous solution of sodium chloride (0.9% saline by weight), obtainable from Baxter Travenol company of Deerfield, IL, is used to saturate the samples.

Equipment

| | |
|---|---|
| Caliper Gauge | Digital gauge with 1 inch (2.54 centimeter) diameter circular foot, such as the Ono Sokki Model GS 503, available from Measure-All, Inc., Fairfield, OH |
| Tensile/compression Tester | Sintech Renew Tensile/Compression running MTS Sintech 3.04 software, available from MTS Systems Corporation of Eden Prairie, MN |
| Load Cell | Suitable for tensile/compression tester, 100N |
| Compression Plate | 1 inch (2.54 centimeter) diameter, flat circular plate attached to crosshead |

Equipment Setup

Gauge Length          4.00 millimeters

Crosshead Speed       5.0 millimeters per minute

Turnaround Distance   One half initial sample caliper

Method

1) Cut a 10 centimeter x 10 centimeter square sample from the material to be tested.

2) Immerse the sample in 0.9% saline solution for 8 minutes.

3) Measure the wet caliper using the caliper gauge under a confining pressure of 0.1 psi (0.7 kPa).

4) Set the turnaround distance (Gauge Length - 1.5 x wet caliper) on the tensile/compression tester.

5) Place the sample on a 140 millimeter diameter flat, circular plate on the tensile/compression tester and start the crosshead.

6) Record the distance at a force of 32 grams (i.e., a pressure of 0.1 psi (0.7 kPa)) on the downward (Downward Distance) and return (Return Distance) cycles of the crosshead.

Calculation

Wet Resiliency = 100*((Gauge Length)-(Return Distance))/((Gauge Length)-(Downward Distance))

While particular embodiments of the present invention have been illustrated and described, it would be obvious to those skilled in the art that various other changes and modifications can be made without departing from the scope of the invention.

**Claims**

**1.**  An absorbent device insertable into the interlabial space of a female wearer, said female wearer having labia and a vestibule floor, said absorbent device having a longitudinal centerline (L), a lateral centerline, and a body contacting surface, said absorbent device comprising a main absorbent portion (22) having a top surface lying in the same plane as said body contacting surface, wherein said top surface faces toward said female wearer's vestibule floor when said absorbent device is worn, said main absorbent portion comprising:

an acquisition member (26) disposed along said longitudinal centerline (L) and extending downwardly from said top surface, said acquisition member having first pores with a first pore volume distribution; and

a storage member (28) in fluid communication with said acquisition member (26), wherein said storage member (28) lies laterally outboard of said acquisition member and extends downwardly from said top surface, said storage member (28) having second pores with a second pore volume distribution;

  **characterized in that**:

said first pore volume distribution is such that at least 75 percent of said first pores have a capillary radius greater than about 20 microns; and

the mean capillary radius of said second pore volume distribution is less than the mean capillary radius of said first pore volume distribution, causing said storage member to draw liquids from said acquisition member (26).

2. An interlabial absorbent device according to Claim 1 wherein said interlabial absorbent device further comprises a pair of flexible extensions which are joined to said main absorbent portion adjacent said top surface thereof.

3. An interlabial absorbent device according to Claims 1 or 2 wherein said acquisition member comprises a first fibrous assembly.

4. An interlabial absorbent device according to Claims 1 or 2 wherein said acquisition member comprises a foam.

5. An interlabial absorbent device according to any of the above claims wherein said storage member comprises a second fibrous assembly.

6. An interlabial absorbent device according to Claim 5 wherein said fibrous assembly comprises a blend of fibers and particulate superabsorbent.

7. An interlabial absorbent device according to any of the above claims wherein said first pore volume distribution is such that at least about 75% of said first pores have a capillary radius greater than about 30 microns.

8. An interlabial absorbent device according to Claim 1 wherein:

said acquisition member has first and second spaced apart sides;

said storage member comprises first and second portions, said first portion of said storage member lying laterally outboard of said first portion of said first side of said acquisition member and said second portion of said storage member lying laterally outboard of said second side of said acquisition member; and

said acquisition member has first pores with a first pore volume distribution such that at least 75 percent of said first pores have a capillary radius greater than about 50 microns.

9. An interlabial absorbent device according to Claim 8 wherein said absorbent device further comprises a pair of flexible extensions joined to said main portion adjacent said top surface thereof, wherein both said main absorbent portion and said flexible extensions have a longitudinal length and said longitudinal length of said main absorbent portion is less than said longitudinal length of said flexible extensions.

**Patentansprüche**

1. Absorbierender Artikel, der in den Raum zwischen den Schamlippen einer Trägerin, die Schamlippen und einen Scheidenvorhofboden besitzt, einschiebbar ist, wobei der absorbierende Artikel eine Längszentrallinie (L), eine Querzentrallinie und eine den Körper berührende Oberfläche aufweist, wobei der absorbierende Artikel einen Hauptabsorptionsteil (22), der eine obere Oberfläche, die in derselben Ebene wie die den Körper berührende Oberfläche liegt, aufweist, wobei die obere Oberfläche zum Scheidenvorhofboden der Trägerin zeigt, wenn der absorbierende Artikel getragen wird, wobei der Hauptabsorptionsteil folgendes umfaßt:

ein Aufnahmeelement (26), das entlang der Längszentrallinie (L) angeordnet ist, und das sich von der oberen Oberfläche nach unten erstreckt, wobei das Aufnahmeelement erste Poren mit einer ersten Porenvolumenverteilung aufweist; und
ein Speicherelement (28) in Fluidverbindung mit dem Aufnahmeelement (26), wobei das Speicherelement (28) seitlich außerhalb des Aufnahmeelements liegt und sich von der oberen Oberfläche nach unten erstreckt, und wobei das Speicherelement (28) zweite Poren mit einer zweiten Porenvolumenverteilung aufweist,

**dadurch gekennzeichnet, daß**

die erste Porenvolumenverteilung so ausgebildet ist, daß mindestens 75 Prozent der ersten Poren einen Kapillarrohrradius von mehr als 20 Mikrometer aufweisen; und

der mittlere Kapillarrohrradius der zweiten Porenvolumenverteilung kleiner als der mittlere Kapillarrohrradius der ersten Porenvolumenverteilung ist, was bewirkt, daß das Speicherelement Flüssigkeiten aus dem Aufnahmeelement (26) zieht.

2. Zwischen den Schamlippen angeordneter absorbierender Artikel nach Anspruch 1, wobei der zwischen den Schamlippen angeordnete absorbierende Artikel weiter ein Paar flexibler Erweiterungen, die mit dem Hauptabsorptionsteil neben dessen oberer Oberfläche verbunden sind, umfaßt.

3. Zwischen den Schamlippen angeordneter absorbierender Artikel nach Anspruch 1 oder 2, wobei das Aufnahmeelement einen ersten Faseraufbau umfaßt.

4. Zwischen den Schamlippen angeordneter absorbierender Artikel nach den Ansprüchen 1 oder 2, wobei das Aufnahmeelement einen Schaum umfaßt.

5. Zwischen den Schamlippen angeordneter absorbierender Artikel nach einem der vorhergehenden Ansprüche, wobei das Speicherelement einen zweiten Faseraufbau umfaßt.

6. Zwischen den Schamlippen angeordneter absorbierender Artikel nach Anspruch 5, wobei der Faseraufbau eine Mischung aus Fasern und einem teilchenförmigen Superabsorptionsmittel umfaßt.

7. Zwischen den Schamlippen angeordneter absorbierender Artikel nach einem der obigen Ansprüche, wobei die erste Porenvolumenverteilung so ausgebildet ist, daß mindestens ungefähr 75% der ersten Poren einen Kapillarrohrradius von mehr als ungefähr 30 Mikrometern aufweisen.

8. Zwischen den Schamlippen angeordneter absorbierender Artikel nach Anspruch 1, wobei:

das Aufnahmeelement erste und zweite voneinander beabstandete Seiten aufweist;

das Speicherelement erste und zweite Teile umfaßt, wobei der erste Teil des Speicherelements seitlich außerhalb des ersten Teils der ersten Seite des Aufnahmeelements liegt, und wobei der zweite Teil des Speicherelements seitlich außerhalb der zweiten Seite des Aufnahmeelements liegt; und

das Aufnahmeelement erste Poren mit einer ersten Porenvolumenverteilung, die derart ausgebildet ist, daß mindestens 75 Prozent der ersten Poren einen Kapillarrohrradius von mehr als ungefähr 50 Mikrometer aufweisen, aufweist.

9. Zwischen den Schamlippen angeordneter absorbierender Artikel nach Anspruch 8, wobei der absorbierende Artikel weiter ein Paar flexibler Erweiterungen, die mit dem Hauptteil neben dessen oberer Oberfläche verbunden sind, umfaßt, wobei sowohl der Hauptabsorptionsteil als auch die flexiblen Erweiterungen eine in Längsrichtung verlaufende Länge aufweisen, und die in Längsrichtung verlaufende Länge des Hauptabsorptionsteils kleiner als die in Längsrichtung verlaufende Länge der flexiblen Erweiterungen ist.

## Revendications

1. Dispositif absorbant pouvant être inséré dans l'espace interlabial d'une utilisatrice, ladite utilisatrice ayant des lèvres et un plancher de vestibule, ledit dispositif absorbant ayant une ligne médiane longitudinale (L), une ligne médiane centrale, et une surface de contact avec le corps, ledit dispositif absorbant comprenant une partie absorbante principale (22) ayant une surface supérieure se trouvant dans le même plan que ladite surface de contact avec le corps, ladite surface supérieure étant dirigée vers le plancher de vestibule de ladite utilisatrice lorsque le dispositif absorbant est porté, ladite partie absorbante principale comprenant :

un élément de recueil (26) disposé le long de ladite ligne médiane longitudinale (L) et s'étendant vers le bas à partir de ladite surface supérieure, ledit élément de recueil ayant des premiers pores, selon une première distribution volumique de pores ; et

un élément de stockage (28) en communication fluidique avec ledit élément de recueil (26), ledit élément de stockage (28) se trouvant à l'extérieur dudit élément de recueil, dans la direction latérale, et s'étendant vers le bas à partir de ladite surface supérieure, ledit élément de stockage (28) ayant des seconds pores, selon une seconde distribution volumique de pores ;

**caractérisé en ce que**

ladite première distribution volumique de pores est telle qu'au moins 75 % desdits premiers pores ont un rayon de capillaire supérieur à environ 20 µm ; et

le rayon de capillaire moyen de ladite seconde distribution volumique de pores est plus petit que le rayon de capillaire moyen de ladite première distribution volumique de pores, ce qui entraîne ledit élément de stockage (28) à aspirer les liquides depuis ledit élément de recueil (26).

**2.** Dispositif absorbant interlabial selon la revendication 1, dans lequel ledit dispositif absorbant interlabial comprend, en outre, une paire d'extensions flexibles, qui sont réunies à ladite partie absorbante principale en contiguïté de ladite surface supérieure de cette dernière.

**3.** Dispositif absorbant interlabial selon la revendication 1 ou 2, dans lequel ledit élément de recueil comprend un premier ensemble fibreux.

**4.** Dispositif absorbant interlabial selon la revendication 1 ou 2, dans lequel ledit élément de recueil comprend une mousse.

**5.** Dispositif absorbant interlabial selon l'une quelconque des revendications précédentes, dans lequel ledit élément de stockage comprend un second ensemble fibreux.

**6.** Dispositif absorbant interlabial selon la revendication 5, dans lequel ledit ensemble fibreux comprend un mélange de fibres et de superabsorbant particulaire.

**7.** Dispositif absorbant interlabial selon l'une quelconque des revendications précédentes, dans lequel ladite première distribution volumique de pores est telle qu'au moins environ 75 % desdits premiers pores ont un rayon de capillaire supérieur à environ 30 µm.

**8.** Dispositif absorbant interlabial selon la revendication 1, dans lequel :

ledit élément de recueil a un premier et un deuxième côtés espacés l'un de l'autre ;

ledit élément de stockage comprend des première et deuxième parties, ladite première partie dudit élément de stockage se trouvant à l'extérieur de ladite première partie dudit premier côté dudit élément de recueil, dans la direction latérale, et ladite deuxième partie dudit élément de stockage se trouvant latéralement à l'extérieur de ladite deuxième partie dudit deuxième côté dudit élément de recueil, dans la direction latérale ; et

ledit élément de recueil possède des premiers pores selon une première distribution volumique de pores, qui est telle qu'environ 75 % desdits premiers pores ont un rayon de capillaire supérieur à environ 50 µm.

**9.** Dispositif absorbant interlabial selon la revendication 8, dans lequel ledit dispositif absorbant comprend une paire d'extensions flexibles, réunies à ladite partie principale en contiguïté de ladite surface supérieure de cette dernière, ladite partie absorbant principale et lesdites extensions flexibles ayant chacune une certaine dimension longitudinale, ladite dimension longitudinale de ladite partie absorbante principale étant plus petite que ladite dimension longitudinale desdites extensions flexibles.

Fig. 1

Fig. 2

Fig. 3

Fig. 4

Fig. 5

Fig. 6

Fig. 7

Fig. 8